# EUROPEAN PATENT APPLICATION

(11) **EP 4 595 930 A1**
(43) Date of publication of application: **06.08.2025**
(21) Application number: 24155067.2
(22) Date of filing: 31.01.2024
(51) Int. Cl.: A61F 2/54, A61F 2/50

(54) **TRANSRADIAL PROSTHESIS**

(71) Applicant: Appsocial.org Stiftung, 8032 Zürich (CH)
(72) Inventor: Trojan, Andreas, 8032 Zürich (CH)
(74) Representative: Keller Schneider Patent- und Markenanwälte AG

(57) **Abstract**

The invention relates to a transradial prosthesis (1) for an upper extremity of a human user, the transradial prosthesis comprising (i) an elbow portion (2) for attaching the transradial prosthesis at an elbow of the upper extremity, (ii) a coupling portion (3) at a distal end of the transradial prosthesis with respect to the elbow portion, and (iii) an intermediate portion (4) between the elbow portion and the coupling portion, wherein the intermediate portion is embodied such that it can be translationally extended along a first direction (5) between the elbow portion and the coupling portion.

## Description

### Technical field

The invention relates to a transradial prosthesis for an upper extremity of a human user.

### State of the art

Transradial prostheses as known in the prior art typically require extensive customization to the anatomy of a specific human. Such prostheses are typically designed for adults and are often not suitable for children. Such prostheses are further often also rather expensive and therefore not accessible to many people for financial reasons, including both adults and children, who would in fact be in need of transradial prostheses.

Prostheses as known in the prior art can be subdivided into passive cosmetic prostheses, passive functional prostheses, externally powered prostheses and body-powered prostheses. While passive functional prostheses may be adapted for widely different tasks, such passive functional prostheses also typically lack simple customization.

### Summary of the invention

It is an object of the invention to create a transradial prosthesis that mitigates at least some of the disadvantages of transradial prostheses as known in the prior art.

The solution of the invention is specified by the features of claim 1.

According to a first aspect of the invention, the invention relates to a transradial prosthesis for an upper extremity of a human user, the transradial prosthesis comprising (i) an elbow portion for attaching the transradial prosthesis at an elbow of the upper extremity, (ii) a coupling portion at a distal end of the transradial prosthesis with respect to the elbow portion, and (iii) an intermediate portion between the elbow portion and the coupling portion, wherein the intermediate portion is embodied such that it can be translationally extended along a first direction between the elbow portion and the coupling portion.

An arm of a human typically comprises both upper arm and forearm, and a forearm typically comprises a radial bone running from wrist to elbow. A transradial prosthesis according to the invention is for an upper extremity, i.e. an arm, of a human ending at some point between the elbow and wrist, i.e. for an arm without hand and wrist and with a shortened forearm and an elbow.

The transradial prosthesis according to the invention comprises an elbow portion that may be used for attaching the transradial prosthesis at an elbow, a coupling portion and an intermediate portion between the elbow portion and the coupling portion. When the transradial prosthesis is attached to the upper extremity of a human, the human's elbow is attached at the elbow portion to the transradial prosthesis, while the intermediate portion extends at least partly along the stump of the forearm. When the transradial prosthesis is attached to the upper extremity of a human, the coupling portion may therefore be positioned at a place where in usual circumstances a wrist would be present.

The intermediate portion is attached in such a way to the elbow portion and to the coupling portion that a translational extension of the intermediate portion changes a distance between the elbow portion and the coupling portion: when the intermediate portion is shortened, the distance between the elbow portion and the coupling portion decreases, and when the intermediate portion is prolonged, the distance between the elbow portion and the coupling portion increases.

Due to the translational extendibility of the intermediate portion, the transradial prosthesis according to the invention may be advantageously adapted to stumps of different extents. This way, a cumbersome customization process of a transradial prosthesis to a specific human as known in the prior art may be simplified and accelerated. The transradial prosthesis according to the invention may further be easily adapted to a changing anatomy: if a child's forearm grows during development, the transradial prosthesis may easily be re-adapted to the changing anatomy. A user of the transradial prosthesis may advantageously also be able to extend the intermediate portion translationally to adapt the transradial prosthesis to different needs in different circumstances: in situations where a tight control of the transradial prosthesis is required, for example, the intermediate portion may be translationally shortened to match a main length of the stump so that the coupling portion is close to an end of the stump, and in situations where a greater reach is required, for example, the intermediate portion may be translationally prolonged so that the coupling portion is further away from the stump.

The intermediate portion may comprise a stiff portion made of a stiff material, which stiff portion is adapted in such a way that forces acting on the coupling portion are guided towards the elbow portion so that the coupling portion is held in place. The stiff portion may in particular coincide with a linear slide enabling translational extendibility of the intermediate portion. The stiff portion may only partially enclose the stump of the forearm to which the transradial prosthesis is attached. In case that the intermediate portion only comprises the stiff portion, for example, the stump of the forearm may therefore be only partially enclosed by the transradial prosthesis. Advantageously, the transradial prosthesis may therefore be lighter, thereby reducing material need, and, depending on external temperature conditions, more comfortable to wear for a human user.

The intermediate portion may also comprise a non-stiff portion in addition to the stiff portion, for example made from a non-stiff textile material. An outer shaft part of the intermediate portion may be at least partly made from a non-stiff textile material, for example. Alternatively, the intermediate portion may only comprise a non-stiff portion and no stiff portion.

In an embodiment of the transradial prosthesis according the first aspect of the invention, the intermediate portion is embodied such that, when the transradial prosthesis is attached to the upper extremity, the intermediate portion at least partially encloses a stump of a forearm of the upper extremity.

In a further embodiment of the transradial prosthesis according to the first aspect of the invention, the intermediate portion comprises a linear slide comprising a first sliding part and a second sliding part, with the first sliding part and the second sliding part being translationally movable with respect to one another.

The linear slide may be arranged in such a way on the transradial prosthesis that the linear slide is positioned in a vicinity of a condyle of the humerus of the upper extremity when the transradial prosthesis is attached to an upper extremity of a human user. The linear slide may be embodied in such a way that it is positioned on substantially only one side of the stump of the forearm when attached.

In a further embodiment of the transradial prosthesis according to the first aspect of the invention, the linear slide is embodied as a sliding contact bearing.

In a further embodiment of the transradial prosthesis according to the first aspect of the invention, the first sliding part is connected to the elbow portion and the second sliding part is connected to the coupling portion.

The connections between the first sliding part to the elbow portion and between the second sliding part and the coupling portion may be embodied as sufficiently stiff so that forces acting on the coupling portion may be guided to the elbow portion so that the coupling portion is held in place.

In a further embodiment of the transradial prosthesis according to the first aspect of the invention, the elbow portion comprises a first elbow part and a second elbow part that are arranged such that, when the transradial prosthesis is attached to the upper extremity, the elbow is stuck between the first elbow part and the second elbow part, and wherein the first elbow part and the second elbow part at least partly enclose an elbow region in which the elbow is positioned when the transradial prosthesis is attached to the upper extremity.

The elbow portion may therefore comprise two parts between which an empty elbow region is arranged. When the transradial prosthesis is attached, an elbow of a human user may be arranged in the elbow region, and the elbow may be clamped between the first elbow part and the second elbow part.

In a further embodiment of the transradial prosthesis according to the first aspect of the invention, the first elbow part and the second elbow part are spaced apart from one another along a second direction, wherein the second direction is substantially orthogonal to the first direction, wherein the first elbow part and the second elbow part are connected to each other by a connection bridge of the transradial prosthesis, wherein the connection bridge is elastically deformable in at least the second direction in such a way that the elbow portion can be attached at elbows of differing sizes, and wherein the elbow region is further at least partly enclosed by the connection bridge.

The connection bridge of the transradial prosthesis connecting the first elbow part to the second elbow part may be made in such a way that the first elbow part and the second elbow part may be elastically distanced from each other. In a not-deformed state of the connection bridge, the transradial prosthesis may be dimensioned such that the elbow region between the first elbow part and the second elbow part is too small to accommodate human elbows of typical sizes. The elastic deformability of the connection bridge may then allow a reversible distancing between the first elbow part and the second elbow part, thereby enlarging the elbow region. Once an elbow is positioned in the elbow region, an elastic restoring force would then clamp the elbow between the first elbow part and the second elbow part. Advantageously, in this way the transradial prosthesis may be attached in a simple manner at the elbow of a human user.

In a further embodiment of the transradial prosthesis according to the first aspect of the invention, the first elbow part comprises a first front segment and a first indented contact segment that is recessed with respect to the first front segment, and the second elbow part comprises a second front segment and a second indented contact segment that is recessed with respect to the second front segment, wherein the first indented contact segment and the second indented contact segment, when the transradial prosthesis is attached to the upper extremity, are in direct contact with the elbow, and wherein the first indented contact segment and the second indented contact segment are recessed away from the elbow region.

The first front segment and the first indented contact segment may provide cushioning functionality. The second front segment and the second indented contact segment may provide cushioning functionality as well. When the transradial prosthesis is attached to the upper extremity, wearing comfort may advantageously be thus increased for the human user. Shapes of the first indented contact segment and of the second indented contact segment may be adapted to typical geometries of an elbow joint. This way, contact surfaces between the elbow and the first elbow part and between the elbow and the second elbow part may thus be increased which advantageously may allow improved attachment of the transradial prosthesis to the elbow.

In a further embodiment of the transradial prosthesis according to the first aspect of the invention, the first indented contact segment is symmetrically positioned with respect to the first front segment, and/or the second indented contact segment is symmetrically positioned with respect to the second front segment.

In a further embodiment of the transradial prosthesis according to the first aspect of the invention, the first indented contact segment is asymmetrically positioned with respect to the first front segment, and/or the second indented contact segment is asymmetrically positioned with respect to the second front segment.

The terms symmetrically positioned and asymmetrically positioned may be defined as follows: the first indented contact segment is symmetrically positioned with respect to the first front segment if their respective geometric centres, projected onto a common plane along which the first front segment is aligned, substantially coincide; and asymmetrically positioned in case their projected geometric centres are spaced apart. An analogous definition may be used for the second indented contact segment and the second front segment.

An asymmetrically positioned first indented contact segment and/or an asymmetrically positioned second indented contact segment may allow improved customisability of the transradial prosthesis in case the first indented contact segment and/or the second indented contact segment are rotatable with respect to the remaining parts of the first elbow part and/or of the second elbow part respectively. In case the first indented contact segment and/or the second indented contact segment are thus movable, their relative positions in the first elbow part and/or in the second elbow part respectively may be altered and thus advantageously be adapted to individual anatomies of human users.

In a further embodiment of the transradial prosthesis according to the first aspect of the invention, the first indented contact segment is embodied in the shape of a first truncated elliptic cone, wherein a base of the first truncated elliptic cone is connected to the first front segment, and the second indented contact segment is embodied in the shape of a second truncated elliptic cone, wherein a base of the second truncated elliptic cone is connected to the second front segment.

In a further embodiment of the transradial prosthesis according to the first aspect of the invention, the elbow portion comprises a fastening part that is embodied such that an upper arm portion is attachable to the elbow portion through interaction with the fastening part.

The transradial prosthesis may be embodied in such a way that an upper arm portion may be attached to the transradial prosthesis. The transradial prosthesis may thus advantageously provide further customizability. The transradial prosthesis and the upper arm portion may be designed individually or jointly in such a way that the upper arm portion is detachably attachable to the transradial prosthesis. The fastening part may preferentially embodied in such a way that the transradial prosthesis and the attached upper arm portion may be rotated to one another around at least one upper arm axis of rotation; the upper arm axis of rotation may be parallel to the second direction, for example.

In a further embodiment of the transradial prosthesis according to the first aspect of the invention, the fastening part comprises two holes, wherein a first hole of the two holes passes centrally, and in particular parallel to the second direction, through the first elbow part, and wherein a second hole of the two holes passes centrally, and in particular parallel to the second direction, through the second elbow part.

The first hole in particular may connect an outside region on the outside of the transradial prosthesis to a first inside of the first indented contact segment, in particular embodied as the first truncated elliptic cone, and the second hole in particular may connect the outside region to a second inside of the second indented contact segment, in particular embodied as the second truncated elliptic cone.

The upper arm portion may be connected using two buttons passing through the first hole and the second hole respectively.

In a further embodiment of the transradial prosthesis according to the first aspect of the invention, the fastening part of the elbow portion comprises a first part of a bayonet mount, which first part of the bayonet mount is configured to interact with a second part of the bayonet mount on the upper arm portion.

The transradial prosthesis may also be designed in such a way that the upper arm portion can be attached to the transradial prosthesis using bayonet mount means.

In a further embodiment of the transradial prosthesis according to the first aspect of the invention, the first elbow part comprises at least two first recessed grips, wherein the first front segment and the first indented contact segment are rotatable about a first axis of rotation by means of the at least two first recessed grips, and the second elbow part comprises at least two second recessed grips, wherein the second front segment and the second indented contact segment are rotatable about a second axis of rotation by means of the at least two second recessed grips.

The at least two first recessed grips may be used for holding and rotating the first front segment and the first indented contact segment; alternatively, the transradial prosthesis may be such that only the first indented contact segment may be rotated using the at least two first recessed grips. The at least two second recessed grips may be used for holding and rotating the second front segment and the second indented contact segment; alternatively, the transradial prosthesis may be such that only the second indented contact segment may be rotated using the at least two second recessed grips.

In a further embodiment of the transradial prosthesis according to the first aspect of the invention, the intermediate portion comprises an outer shaft part, in particular with a lacing system, which outer shaft part, when the transradial prosthesis is attached to the upper extremity, encloses the stump of the forearm of the upper extremity, and wherein the outer shaft part is attached to, in particular glued to, the linear slide.

The outer shaft part may enclose the stump of the forearm when attached. The outer shaft part may advantageously be made of flexible and stretchable materials that adapt their shape to the anatomy of the user when the transradial prosthesis is attached. The outer shaft part may comprise waterproof/breathable fabrics. Flexible and stretchable materials may further improve the customizability of the transradial prosthesis: in case the intermediate portion is prolonged, for example, the outer shaft part may be stretched and still cover the user's forearm when stretched. The linear slide is preferentially formed in such a way that it comprises a sufficiently large surface onto which the outer shaft part may be glued.

In a further embodiment of the transradial prosthesis according to the first aspect of the invention, the transradial prosthesis comprises at least one of the following materials: rubber, ethylene-vinyl acetate, polypropylene, polyether block amide, polyester, polyamide, cotton, silicone, memory foam, wherein in particular the first front segment, the first indented contact segment, the second front segment and the second indented contact segment are made of silicone and/or memory foam, and/or wherein in particular the outer shaft part is made of polyester fibres, polyamide fibres and/or cotton fibres, and/or wherein in particular the linear slide and the connection bridge are made of rubber and/or ethylene-vinyl acetate by injection moulding.

In a further embodiment of the transradial prosthesis according to the first aspect of the invention, the coupling portion comprises a first coupling portion part and/or a second coupling portion part, with the first coupling portion part facing towards the intermediate portion and/or the second coupling portion part facing away from the transradial prosthesis, and an exchangeable spacer element is attachable to the first coupling portion part, wherein the spacer element is in particular heatable.

Using the first coupling portion part, the transradial prosthesis advantageously may be customized in an improved manner. In case the minimal extent of the transradial prosthesis, which minimal extent may be realized when the first sliding part is as close as possible to the second sliding part, is too large for the stump of a user, the first coupling portion part may be used for attaching a spacer element to the transradial prosthesis, thereby enabling the use of the transradial prosthesis for humans with very short forearms. The second coupling portion part may be used for attaching functional hand replacements/adaptors to the transradial prosthesis. The second coupling portion part may comprise a first part of fastening means, e.g. a first part of bayonet mounting means.

The coupling portion may also only comprise the second coupling portion part.

According to a second aspect of the invention, the invention may relate to a coupling arrangement comprising a coupling portion for a prosthesis, which coupling portion may be used as part of a transradial prosthesis according to the first aspect of the invention. The coupling portion of the coupling arrangement according to the second aspect of the invention may, however, also be used as part of another prosthesis. The coupling portion of the coupling arrangement according to the second aspect of the invention may therefore be used independently of the transradial prosthesis according to the first aspect of the invention. Together with a functional adaptor, the coupling portion may form the coupling arrangement according to the second aspect of the invention.

The second coupling portion part may comprise a first part of a bayonet mount for attaching the functional adaptor to the coupling portion, and the functional adaptor may comprise a second part of the bayonet mount: together, the first part and the second part may provide the bayonet mount. The second coupling portion part may comprise four reception segments angularly spaced by 90 degrees from each other, a locking structure and a swivel. The second part of the bayonet mount on the functional adaptor may comprise a complimentary structure with four insertion segments which may be inserted into the respective four reception segments of the second coupling portion part.

The four reception segments may be provided by four protruding segments. In a first direction, the four protruding segments may have a protruding segment length. The four insertion segments and the protruding segments may be dimensioned such that once the four insertion segments are inserted past the four protruding segments, i.e. deeper into the second coupling portion part than the protruding segment length, the functional adaptor may be rotated. After such a rotation, the four insertion segments may be arranged behind respective protruding segments.

The second coupling portion part may be embodied such that when the swivel is rotated, the locking structure may be displaced along the first direction. To attach a functional adaptor to the coupling portion, the following steps may be carried out: 1) the four insertion segments of the functional adaptor may be inserted into the four reception segments of the second coupling portion part; 2) next, the functional adaptor may be rotated around an axis parallel to the first direction; and 3) next, the swivel of the second coupling portion part may be rotated by 90 degrees around the axis parallel to the first direction. The rotation of the swivel by 90 degrees may displace the locking structure in such a way that the functional adaptor cannot be removed from the coupling portion; to be able to remove the attached functional adaptor, the swivel may need to be rotated again by 90 degrees.

The coupling portion, and specifically the swivel, may advantageously be embodied in such a way that it is visually immediately transparent whether the swivel is in an orientation that allows insertion or removal of a functional adaptor into the coupling portion, or whether the swivel is in an orientation in which the functional adaptor is locked.

Other advantageous embodiments and combinations of features come out from the detailed description below and the entirety of the claims.

### Brief description of the drawings

The drawings used to explain the embodiments show:
- Fig. 1: shows a perspective view of a first embodiment of a transradial prosthesis;
- Fig. 2: shows two side views of the first embodiment of a transradial prosthesis;
- Fig. 3: shows a perspective view of a second embodiment of a transradial prosthesis and two side views through a second coupling portion part;
- Fig. 4: shows two perspective views of an upper arm portion;
- Fig. 5: shows a side view of the second embodiment with an upper arm portion in an attachment position;
- Fig. 6: shows a perspective view and a side view of the second embodiment of a transradial prosthesis with an attached upper arm portion; and
- Fig. 7: shows a perspective view of the second embodiment of a transradial prosthesis with an attached upper arm portion and a functional adaptor.

In the figures, the same components are given the same reference symbols.

### Preferred embodiments

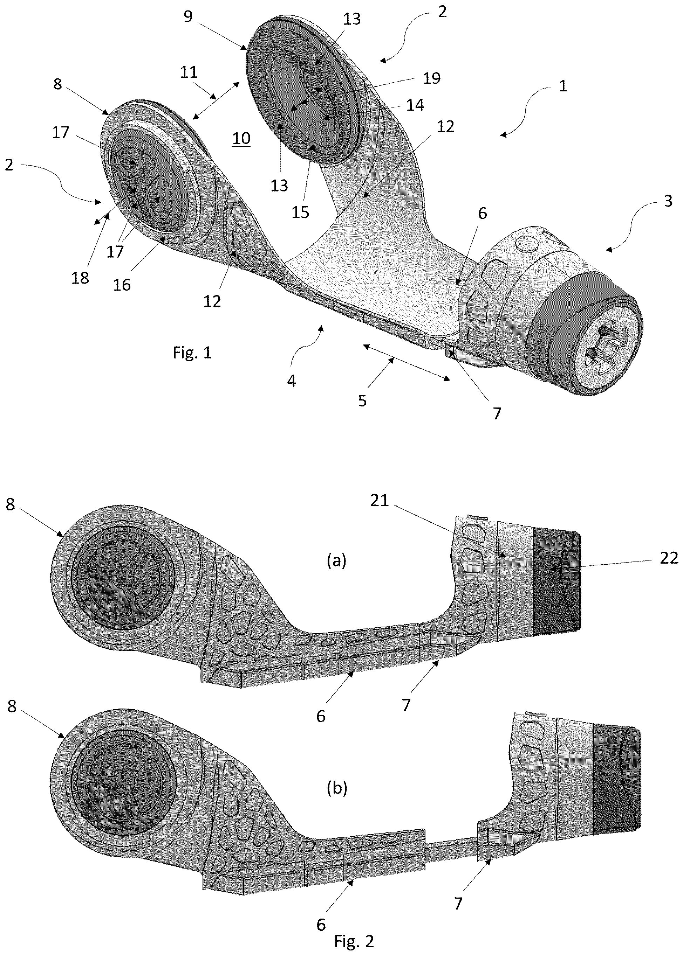

**Fig. 1** shows a perspective view of a first embodiment of a transradial prosthesis 1. The transradial prosthesis 1 comprises (i) an elbow portion 2 comprising a first elbow part 8 and a second elbow part 9 that are connected by a connection bridge 12 to each other, (ii) an intermediate portion 4 comprising a linear slide with a first sliding part 6 and a second sliding part 7, and (iii) a coupling portion 3. The elbow portion 2 is connected to the first sliding part 6, and the coupling portion 3 is connected to the second sliding part 7.

Between the first elbow part 8 and the second elbow part 9, an elbow region 10 is arranged in which elbow region 10 an elbow is positioned when the transradial prosthesis 1 is attached to an arm of a human user. The first elbow part 8 and the second elbow part 9 are spaced apart from one another along a second direction 11 that is orthogonal to a first direction 5 along which the transradial prosthesis 1 can be translationally extended using the linear slide. The connection bridge 12 is elastically deformable in at least the second direction 11: this way, an elastic restoring force may facilitate clamping an elbow between the first elbow part 8 and the second elbow part 9.

The first elbow part 8 comprises a first front segment and a first indented contact segment, and the second elbow part 9 comprises a second front segment 13 and a second indented contact segment 14. The first front segment, the first indented contact segment, the second front segment 13 and the second indented contact segment 14 face the elbow region 10. When the transradial prosthesis 1 is attached to an upper extremity of a human, the elbow touches the first indented contact segment and the second indented contact segment 14. The first indented contact segment and the second indented contact segment 14 are embodied in the form of a truncated elliptic cone, with a base of the truncated elliptic cone connected to the respective front segment: a base 15 of the second indented contact segment 14 is connected to the second front segment 13, for example. On the side of the first elbow part 8 and on the side of the second elbow part 9 facing towards an outside of the transradial prosthesis, i.e. away from the elbow region 10, a first part of mounting means 16 is arranged which may be used to attach an upper arm portion to the transradial prosthesis 1.

The first indented contact segment and the second indented contact segment 14 are asymmetrically positioned with respect to the first front segment and the second front segment 13. The first elbow part 8 and the second elbow part 9 comprise three first recessed grips 17 and three second recessed grips, respectively: the three first recessed grips 17 may be used to rotate the first front segment and the first indented contact segment around a first axis of rotation 18; the three second recessed grips may be used to rotate the second front segment 17 and the second indented contact segment 14 around a second axis of rotation 19. When the transradial prosthesis 1 is not attached to an upper extremity of a human, the first axis of rotation 18 and the second axis of rotation 19 may be parallel to the second direction 11.

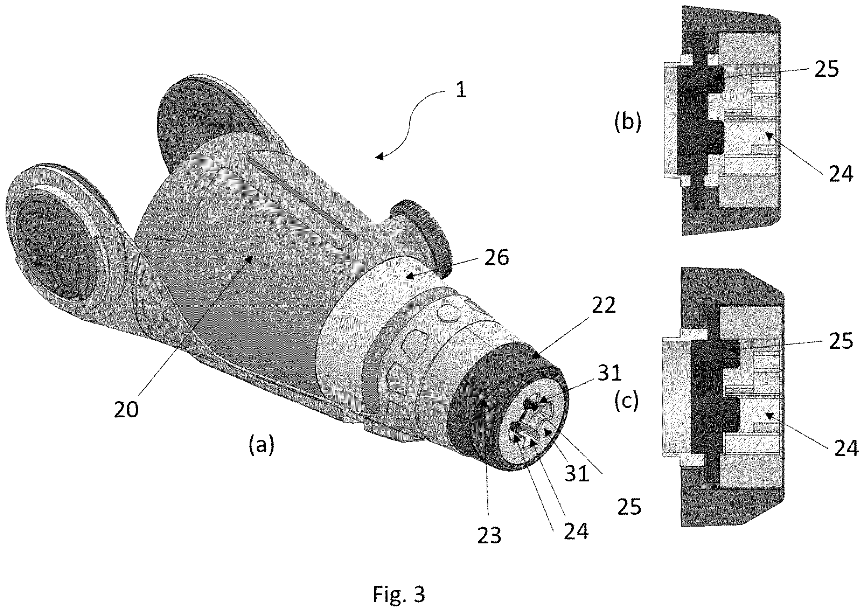

**Fig. 2** shows two side views of the first embodiment of a transradial prosthesis 1. Compared to Fig. 2a, in Fig. 2b the linear slide is extended in the first direction 5. At a first coupling portion part 21 facing towards the elbow portion 2, a spacer may be attached to the transradial prosthesis 1; at a second coupling portion part 22 of the coupling portion 7, a functional adaptor may be attached to the transradial prosthesis 1. The functioning of the second coupling portion part 22 is explained in more detail with reference to Fig. 3.

**Fig. 3** shows a perspective view of a second embodiment of a transradial prosthesis 1 and two side views through a second coupling portion part 22. The second embodiment of a transradial prosthesis 1 is based on the first embodiment shown in Figs. 1 and 2: in addition to the first embodiment, in the second embodiment an outer shaft part 20 is present.

The outer shaft part 20 is glued to the first sliding part 6 and to the second sliding part 7. The outer shaft part 20 may also be attached, in particular glued, to the coupling portion 3. A flexible segment 26 of the outer shaft part 20, which flexible segment 26 may be made of a stretchable material, may facilitate an extension of the transradial prosthesis in the first direction 5. The flexible segment 26 may be arranged above the part of the intermediate portion 4 where the extension may be carried out.

The second coupling portion part 22 comprises a first part of a bayonet mount for attaching a functional adaptor to the coupling portion 3, and the functional adaptor may comprise a second part of the bayonet mount: together, the first part and the second part may provide the bayonet mount. The second coupling portion part 22 comprises four reception segments 24 angularly spaced by 90 degrees from each other, a locking structure 25 and a swivel 23. The second part of the bayonet mount on the functional adaptor may comprise a complimentary structure with four insertion segments which may be inserted into the respective four reception segments 24 of the second coupling portion part 22.

The four reception segments 24 are provided by four protruding segments 31. In the first direction 5, the four protruding segments 31 have a protruding segment length. The four insertion segments and the protruding segments 31 are dimensioned such that once the four insertion segments are inserted past the four protruding segments 31, i.e. deeper into the second coupling portion part 22 than the protruding segment length, the functional adaptor may be rotated. In the embodiment of Fig. 3a, for example, a fully inserted functional adaptor may be rotated in a clockwise direction; after such a rotation, the four insertion segments may be arranged behind respective protruding segments 31.

The second coupling portion part 22 is embodied such that when the swivel 23 is rotated, the locking structure 25 is displaced along the first direction 5. To attach a functional adaptor to the coupling portion 3, the following steps may be carried out: 1) the four insertion segments of the functional adaptor are inserted into the four reception segments 24 of the second coupling portion part 22; 2) next, the functional adaptor is rotated around an axis parallel to the first direction 5; and 3) next, the swivel 23 of the second coupling portion part 22 is rotated by 90 degrees around the axis parallel to the first direction 5.

The rotation of the swivel 23 by 90 degrees displaces the locking structure 25 in such a way that the functional adaptor cannot be removed from the coupling portion 3; to be able to remove the attached functional adaptor, the swivel 23 would need to rotated again by 90 degrees.

In Fig. 3b, the locking structure 25 is further removed from the four reception segments 24 than in Fig. 3c. A functional adaptor which is inserted into the second coupling portion part 22 of Fig. 3b may therefore be rotated after being inserted; after the locking structure 25 has been moved towards the four reception segments 24 as indicated in Fig. 3c by rotating the swivel 23, the inserted functional adaptor may no longer be rotated.

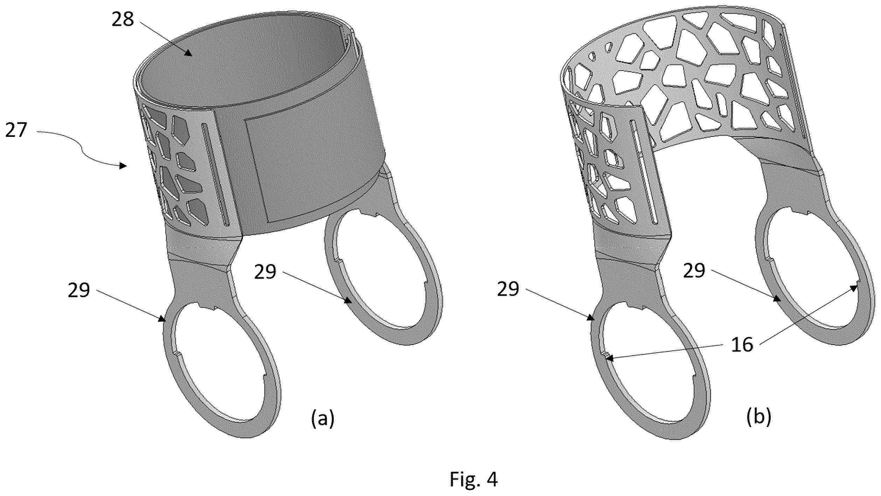

**Fig. 4** shows two perspective views of an upper arm portion 27 that may be attached to a transradial prosthesis 1 as shown Figs. 1 to 3. In Fig. 4a, both a support structure 29 of the upper arm portion 27 as well as a cuff 28 are shown, wherein the cuff 28 may be fixedly attached to the support structure 29. In Fig. 4b, only the support structure 29 of the upper arm portion 27 is shown. The upper arm portion 27 comprises a second part of mounting means 16 that may interact with the first part of mounting means 16 on the elbow portion shown in Figs. 1 to 3.

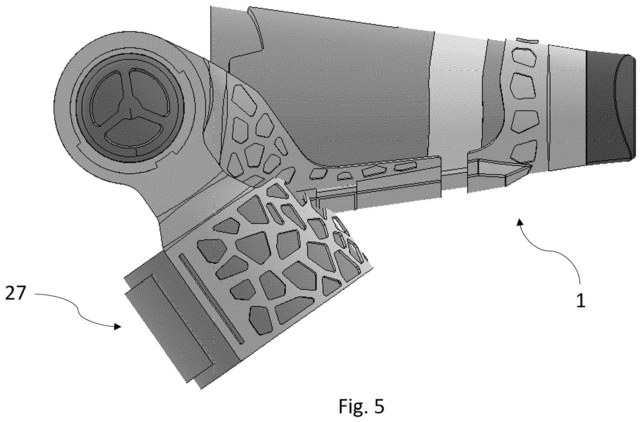

**Fig. 5** shows a side view of the second embodiment of a transradial prosthesis 1 with an upper arm portion 27 in an attachment position. The upper arm portion 27 of Fig. 5 corresponds to the upper arm portion 27 of Fig. 4a. The first part of the mounting means 16 on the elbow portion and the second part of the mounting means 16 on the upper arm portion 27 are embodied in such a way that in order to attach or detach the upper arm portion 27 from the transradial prosthesis 1, the transradial prosthesis 1 needs to be inclined with a sufficiently acute angle with respect to the upper arm portion 27, this relative positioning between the upper arm portion 27 and the transradial prosthesis 1 corresponding to an attachment position. The acute angle at which attachment or detachment may be carried out may correspond to an approximately smallest angle that an average human may obtain between upper arm and forearm. This way, the upper arm portion 27 may not be accidentally detached from the transradial prosthesis 1. Once the transradial prosthesis 1 and the upper arm portion 27 are away from the attachment position, the upper arm portion 27 may not be detached from the transradial prosthesis 1.

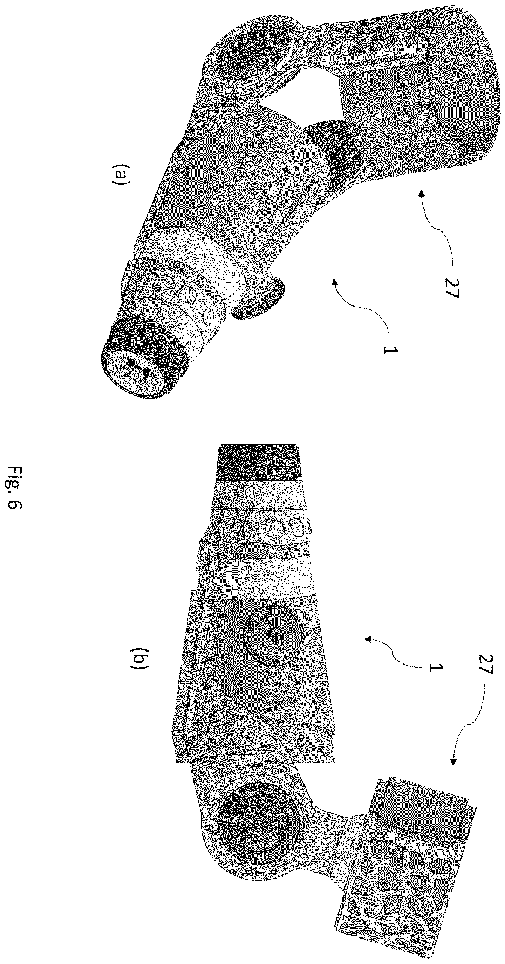

**Fig. 6** shows a perspective view and a side view of the second embodiment of a transradial prosthesis 1 together with an attached upper arm portion 27. The upper arm portion of Fig. 6 corresponds to the upper arm portion of Fig. 4a.

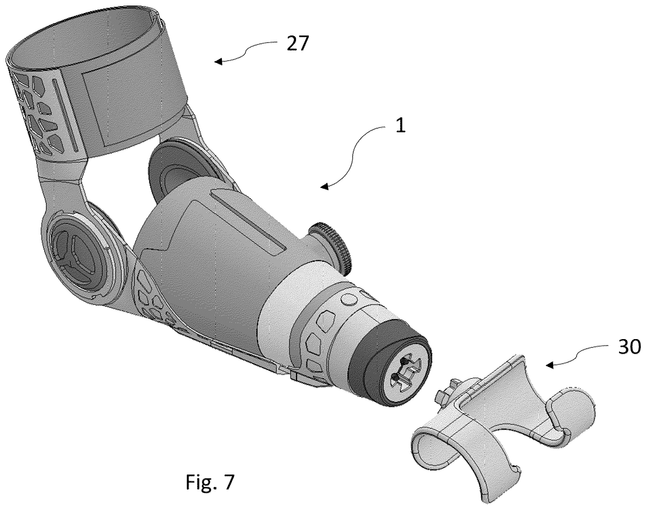

**Fig. 7** shows a perspective view of the second embodiment of a transradial prosthesis 1 with an attached upper arm portion 27. Further, a functional adaptor 30 is shown which may be attached to the coupling portion 3 as described above with reference to Fig. 3.

## Claims

1. Transradial prosthesis (1) for an upper extremity of a human user, the transradial prosthesis (1) comprising (i) an elbow portion (2) for attaching the transradial prosthesis (1) at an elbow of the upper extremity, (ii) a coupling portion (3) at a distal end of the transradial prosthesis (1) with respect to the elbow portion (2), and (iii) an intermediate portion (4) between the elbow portion (2) and the coupling portion (3), wherein the intermediate portion (4) is embodied such that it can be translationally extended along a first direction (5) between the elbow portion (2) and the coupling portion (3).

2. Transradial prosthesis (1) according to claim 1, wherein the intermediate portion (4) is embodied such that, when the transradial prosthesis (1) is attached to the upper extremity, the intermediate portion (4) at least partially encloses a stump of a forearm of the upper extremity.

3. Transradial prosthesis (1) according to claim 1 or 2, wherein the intermediate portion (4) comprises a linear slide comprising a first sliding part (6) and a second sliding part (7), with the first sliding part (6) and the second sliding part (7) being translationally movable with respect to one another.

4. Transradial prosthesis (1) according to claim 3, wherein the linear slide is embodied as a sliding contact bearing.

5. Transradial prosthesis (1) according to claim 3 or 4, wherein the first sliding part (6) is connected to the elbow portion (2) and wherein the second sliding part (7) is connected to the coupling portion (3).

6. Transradial prosthesis (1) according to any one of the preceding claims, wherein the elbow portion (2) comprises a first elbow part (8) and a second elbow part (9) that are arranged such that, when the transradial prosthesis (1) is attached to the upper extremity, the elbow is stuck between the first elbow part (8) and the second elbow part (9), and wherein the first elbow part (8) and the second elbow part (9) at least partly enclose an elbow region (10) in which the elbow is positioned when the transradial prosthesis (1) is attached to the upper extremity.

7. Transradial prosthesis (1) according to claim 6, wherein the first elbow part (8) and the second elbow part (9) are spaced apart from one another along a second direction (11), wherein the second direction (11) is substantially orthogonal to the first direction (5), wherein the first elbow part (8) and the second elbow part (9) are connected to each other by a connection bridge (12) of the transradial prosthesis (1), wherein the connection bridge (12) is elastically deformable in at least the second direction (11) in such a way that the elbow portion (2) can be attached at elbows of differing sizes, and wherein the elbow region (10) is further at least partly enclosed by the connection bridge (12).

8. Transradial prosthesis (1) according to claim 6 or 7, wherein the first elbow part (8) comprises a first front segment and a first indented contact segment that is recessed with respect to the first front segment, and wherein the second elbow part (9) comprises a second front segment (13) and a second indented contact segment (14) that is recessed with respect to the second front segment (13), wherein the first indented contact segment and the second indented contact segment (14), when the transradial prosthesis (1) is attached to the upper extremity, are in direct contact with the elbow, and wherein the first indented contact segment and the second indented contact segment (14) are recessed away from the elbow region (10).

9. Transradial prosthesis (1) according to claim 8, wherein the first indented contact segment is symmetrically positioned with respect to the first front segment, and/or wherein the second indented contact segment (14) is symmetrically positioned with respect to the second front segment (13).

10. Transradial prosthesis (1) according to claim 8, wherein the first indented contact segment is asymmetrically positioned with respect to the first front segment, and/or wherein the second indented contact segment (14) is asymmetrically positioned with respect to the second front segment (13).

11. Transradial prosthesis (1) according to claim 10, wherein the first indented contact segment is embodied in the shape of a first truncated elliptic cone, wherein a base of the first truncated elliptic cone is connected to the first front segment, and wherein the second indented contact segment (14) is embodied in the shape of a second truncated elliptic cone, wherein a base (15) of the second truncated elliptic cone is connected to the second front segment (13).

12. Transradial prosthesis (1) according to any one of the preceding claims, wherein the elbow portion (2) comprises a fastening part that is embodied such that an upper arm portion (27) is attachable to the elbow portion (2) through interaction with the fastening part.

13. Transradial prosthesis (1) according to claim 12, wherein the fastening part of the elbow portion (2) comprises a first part of mounting means (16), which first part of the mounting means (16) is configured to interact with a second part of the mounting means (16) on the upper arm portion (27).

14. Transradial prosthesis (1) according to any one of claims 8 to 13, wherein the first elbow part (8) comprises at least two first recessed grips (17), wherein the first front segment and the first indented contact segment are rotatable about a first axis of rotation (18) by means of the at least two first recessed grips (17), and wherein the second elbow part (9) comprises at least two second recessed grips, wherein the second front segment (13) and the second indented contact segment (14) are rotatable about a second axis of rotation (19) by means of the at least two second recessed grips.

15. Transradial prosthesis (1) according to any one of claims 3 to 14, wherein the intermediate portion (4) comprises an outer shaft part (20), in particular with a lacing system, which outer shaft part (20), when the transradial prosthesis (1) is attached to the upper extremity, encloses the stump of the forearm of the upper extremity, and wherein the outer shaft part (20) is attached to, in particular glued to, the linear slide (6, 7).

16. Transradial prosthesis (1) according to any one of the preceding claims, wherein the transradial prosthesis (1) comprises at least one of the following materials: rubber, ethylene-vinyl acetate, polypropylene, polyether block amide, polyester, polyamide, cotton, silicone, memory foam, wherein in particular the first front segment, the first indented contact segment, the second front segment (13) and the second indented contact segment (14) are made of silicone and/or memory foam, and/or wherein in particular the outer shaft part (20) is made of polyester fibres, polyamide fibres and/or cotton fibres, and/or wherein in particular the linear slide (6, 7) and the connection bridge (12) are made of rubber and/or ethylene-vinyl acetate by injection moulding.

17. Transradial prosthesis (1) according to any one of the preceding claims, wherein the coupling portion (3) comprises a first coupling portion part (21) and/or a second coupling portion part (22), with the first coupling portion part (21) facing towards the intermediate portion (4) and/or the second coupling portion part (22) facing away from the transradial prosthesis (1), and wherein an exchangeable spacer element is attachable to the first coupling portion part (21), wherein the spacer element is in particular heatable.
